**Europäisches Patentamt**

**European Patent Office**

**Office européen des brevets**

(11) Veröffentlichungsnummer: **0 047 356**
**B1**

(12) **EUROPÄISCHE PATENTSCHRIFT**

(45) Veröffentlichungstag der Patentschrift:
**11.07.84**

(21) Anmeldenummer: **81103233.3**

(22) Anmeldetag: **29.04.81**

(51) Int. Cl.³: **A 61 L 2/12**, A 61 L 2/08,
A 61 L 2/20, A 61 L 2/06

(54) **Vorrichtung zum Sterilisieren von medizinischen, insbesondere zahnmedizinischen Gegenständen.**

(30) Priorität: **09.09.80 DE 3033855**

(43) Veröffentlichungstag der Anmeldung:
**17.03.82 Patentblatt 82/11**

(45) Bekanntmachung des Hinweises auf die Patenterteilung:
**11.07.84 Patentblatt 84/28**

(84) Benannte Vertragsstaaten:
**AT CH DE FR GB IT LI**

(56) Entgegenhaltungen:
**DE - A - 2 532 176**
**GB - A - 1 406 789**

(73) Patentinhaber: **SIEMENS AKTIENGESELLSCHAFT,**
**Berlin und München Wittelsbacherplatz 2,**
**D-8000 München 2 (DE)**

(72) Erfinder: **Hohmann, Eugen, Am Leimenberg 32,**
**D-6140 Bensheim (DE)**

# Beschreibung

Die Erfindung bezieht sich auf eine Vorrichtung zum Sterilisieren von medizinischen, insbesondere zahnmedizinischen Gegenständen, wie Handinstrumenten od. dgl., unter Verwendung eines Strahlers mit hoher Energieabstrahlung, vorzugsweise eines IR- oder Mikrowellenstrahlers und eines flüssigen, dampfbildenden Reaktionsmediums.

Die im medizinischen Bereich zum Sterilisieren von Gegenständen, wie Handinstrumenten, Werkzeugen, od. dgl., verwendeten Heissdampfsterilisatoren (Autoklaven) bedingen, unter anderem wegen des relativ grossen Kammervolumens, relativ lange Erwärmungs- und Abkühlzeiten. Der für eine Sterilisation anzusetzende Zeitaufwand ist deshalb und insbesondere dann, wenn nur wenige Gegenstände, z.B. nur die für eine Behandlung gebrauchten Instrumente, sterilisiert werden sollen, unangemessen hoch. Ausserdem ist der apparative Aufbau eines solchen Autoklaven relativ aufwendig. Letzteres trifft auch für eine andere bekannte Einrichtung (GB-A1-1406789) zu, die nach dem Mikrowellensterilisationsverfahren arbeitet. Der dort beschriebene Apparat enthält ein den bekannten Mikrowellenherden ähnliches metallisches Gehäuse, in dem ein mehrere Gefache zur Aufnahme der zu sterilisierenden Gegenstände aufnehmender, gasdichter Behälter aus Mikrowellen durchlassendem Material angeordnet werden kann. Die von einem ausserhalb des den Behälter aufnehmenden Raumes angeordneten Magnetron erzeugte Mikrowellenstrahlung wird über einen Führungskanal einem langsam sich drehenden Flügelrad zugeführt, das die Strahlung im Gehäuse verteilt. Um im Behälter die für eine optimale Sterilisation notwendige Luftfeuchtigkeit zu bekommen, ist in dem Behälter noch ein kleiner Flüssigkeitsbehälter vorgesehen.

Aufgabe der Erfindung ist es, eine Vorrichtung anzugeben, die einfacher aufgebaut ist und mit der die Sterilisationszeit im Vergleich zu herkömmlichen Vorrichtungen wesentlich reduziert werden kann.

Die gestellte Aufgabe wird gemäss der Erfindung dadurch gelöst, dass für die zu sterilisierenden Gegenstände ein relativ kleiner Aufnahmebehälter und ein mit diesem in Verbindung stehender, das Reaktionsmedium aufnehmender weiterer Behälter mit einem im Vergleich zum Volumen des Aufnahmebehälters sehr kleinen Behältervolumen vorgesehen sind, und dass der weitere Behälter im Bereich höchster Energiedichte des das Reaktionsmedium verdampfenden Strahlers angeordnet ist.

Im Gegensatz zu der obengenannten Einrichtung wird das Sterilisiergut ausserhalb des vom Strahler erzeugten Feldes gehalten; diese Teile werden also nur von dem in dem weiteren Behälter erzeugten Dampf beaufschlagt. Besonders vorteilhaft ist die Verwendung eines Mikrowellenstrahlers. Mit Hilfe der Mikrowellenenergie können nämlich auch solche Mikroorganismen sicher abgetötet werden, die an sich nicht flüssigkeitsgefüllt sind, z.B. solche, wie sie in Sporenbildnern anzutreffen sind. Weitere Vorzüge sind, dass die normalerweise bei Einwirkung von Mikrowellenenergie auf Metallteile bestehende Funkenstreckenbildung an Stosskanten, Trennfugen usw., die eine Oberflächenzerstörung des zu sterilisierenden Gegenstandes zur Folge haben würde, bei Einsatz einer dampfbildenden Flüssigkeit vermieden wird. Die ausserdem normalerweise nicht in Fugen und Ritzen eindringenden und dadurch die dort befindlichen Mikroorganismen nicht abtötenden Mikrowellen führen in Verbindung mit dem Reaktionsmedium jedoch zu dem gewünschten Erfolg.

Hält man den Behälter zur Aufnahme der zu sterilisierenden Gegenstände, also den Sterilisationsraum, so klein wie möglich (vorteilhafterweise in der Grösse, die der Aufnahme nur eines oder nur einiger weniger zu sterilisierender Gegenstände entspricht), dann ist die zur Dampferzeugung erforderliche Flüssigkeitsmenge relativ klein, wodurch eine extrem kurze Aufheizzeit erzielt wird. Im gleichen Masse verkürzt sich dann auch die Abkühlzeit.

Bei einer Behältergrösse von etwa ≥ 50 ml zur Aufnahme des Sterilisiergutes, einer Reaktionsmediumsmenge von ca. 4 ml und einer Strahlerleistung von etwa 500 W ist mit der vorgeschlagenen Vorrichtung eine Sterilisation in wenigen Minuten möglich. Durch die Wahl geeigneter Reaktionsmedien mit unterschiedlichen Siedpunkten kann eine rasche und sichere, auf das betreffende Material abgestellte Sterilisation erreich werden.

Vorteilhafte Ausgestaltungen und Weiterbildungen der Erfindung sind in den Unteransprüchen enthalten.

Mehrere Ausführungsbeispiele der Erfindung werden nachfolgend anhand der Zeichnung erläutert. Es zeigen:

Fig. 1 die erfindungsgemässe Vorrichtung in einer Prinzipdarstellung im Schnitt,

Fig. 2 einen Querschnitt entlang der Linie II/II in Fig. 1,

Fig. 3 ein anderes Ausführungsbeispiel der erfindungsgemässen Vorrichtung ebenfalls in einer Prinzipdarstellung teilweise im Schnitt,

Fig. 4 ein anderes Ausführungsbeispiel vom Behälter zur Aufnahme der zu sterilisierenden Gegenstände und des Reaktionsmediums,

Fig. 5 einen Einlegebeutel mit Reaktionsflüssigkeit.

Die erfindungsgemässe Vorrichtung enthält einen in einem Gehäuse 1 leicht herausnehmbar gehalterten, flaschenförmig ausgebildeten Behälter 2, der durch seine Formgebung einen ersten Behälterabschnitt 2a zur Aufnahme der zu sterilisierenden Gegenstände, z.B. eines mit 3 bezeichneten zahnärztlichen Instruments, und einen zweiten Behälterabschnitt 2b im flaschenhalsartig sich stark verjüngenden Teil zur Aufnahme eines flüssigen Reaktionsmediums 4 bildet.

Der Aufnahmebehälter 2a ist nach oben hin durch einen Schraubdeckel 5 mit dazwischengelegter Dichtung 6 druckfest abgeschlossen, nach unten hin zum Behälterabschnitt 2b über einen gelochten Einsatz 7 für einen Dampfdurchtritt offen.

Der Einsatz 7 verhindert, dass insbesondere kleinere zu sterilisierende Gegenstände in den unteren Behälterabschnitt 2b mit der Reaktionsflüssigkeit fallen können.

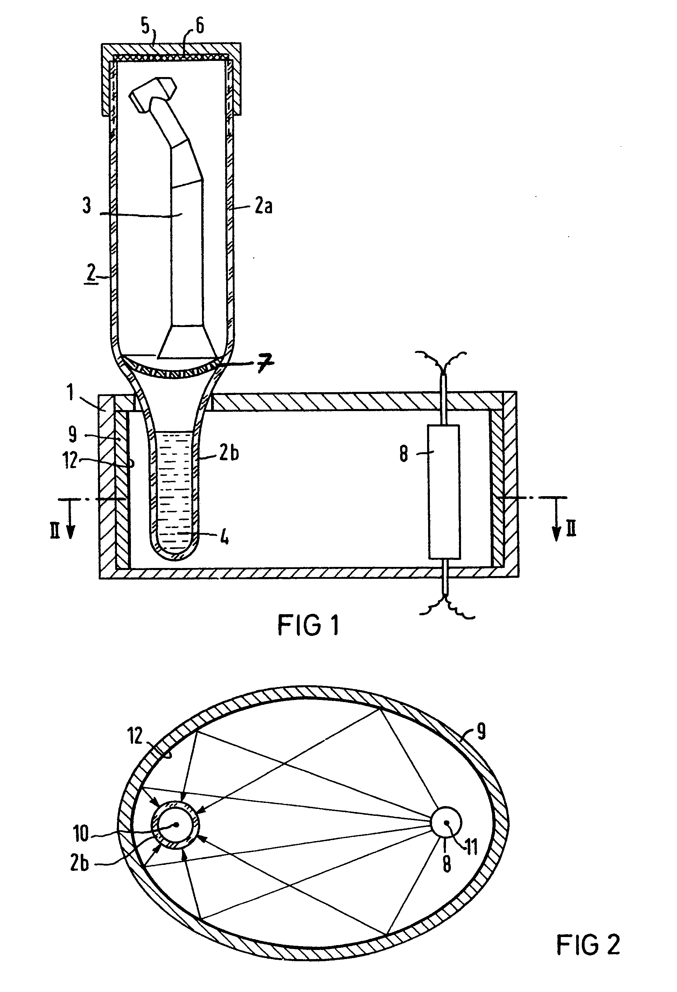

Im Gehäuse, in das der untere Behälterabschnitt 2b eintaucht, sind eine Strahlungsquelle in Form eines stabförmigen Infrarotstrahlers 8 sowie ein elliptischer Reflektor 9 angeordnet. Der flaschenhalsförmige Behälterteil 2b zur Aufnahme des Reaktionsmediums 3 befindet sich im ersten Brennpunkt (Pos. 10) des im Querschnitt elliptisch ausgebildeten Reflektors 9 (vgl. Fig. 2), in dessen zweiten Brennpunkt (Pos. 11) der stabförmige IR-Strahler 8 angeordnet ist. Wie durch einige, mit Pfeilen versehene Strahlungslinien angedeutet, wird durch die spezielle Anordnung eine sehr hohe Strahlungsverdichtung im Bereich des Behälters 2b erzielt, der zu einer sehr raschen Verdampfung des Reaktionsmediums führt.

Zweckmässigerweise entspricht die Höhe des Reflektors 9 in etwa der Höhe des Behälterabschnittes 2b, in dem sich das Reaktionsmedium befindet. Der Reflektor 9 besteht vorteilhafterweise aus dünnwandigem Material mit geringer Wärmekapazität und kann, wie in Fig. 1 mit der Pos. 12 angedeutet, mit einem gut reflektierenden Belag, z.B. mit einer Goldbeschichtung, versehen sein. Die Länge des stabförmigen IR-Strahlers 8 ist vorteilhafterweise nur geringfügig kleiner als die Bauhöhe des Reflektors, wodurch ein möglichst grosses Feld erzeugt wird. Bei einem angenommenen Volumen des Aufnahmebehälters 2a für das zu sterilisierende Gut von etwa 50 ml und einem Volumen von etwa 4 ml für den Behälterabschnitt 2b genügt eine Strahlerleistung von etwa 500 W. Der Infrarotstrahler kann direkt mit Netzspannung betrieben werden. Um eine möglichst verlustlose Umsetzung der Energie zu erzielen, besteht wenigstens der Behälterabschnitt 2b zur Aufnahme des Raktionsmediums aus einem Werktoff mit guter Infrarotdurchlässigkeit, z.B. aus Glas. Vorteilhafterweise ist die Strahlung des Infrarotstrahlers 8 spektral an die Absorption des verwendeten Reaktionsmediums angepasst, d.h. Absorption und Strahlungsintensitätskurven sind über den Strahlungsbereich gesehen etwa deckungsgleich. Als Reaktionsmedium können verschiedene Flüssigkeiten oder Flüssigkeitsgemische verwendet werden, die eine hohe Infrarotabsorption aufweisen, z.B. Wasser, Alkohole usw.

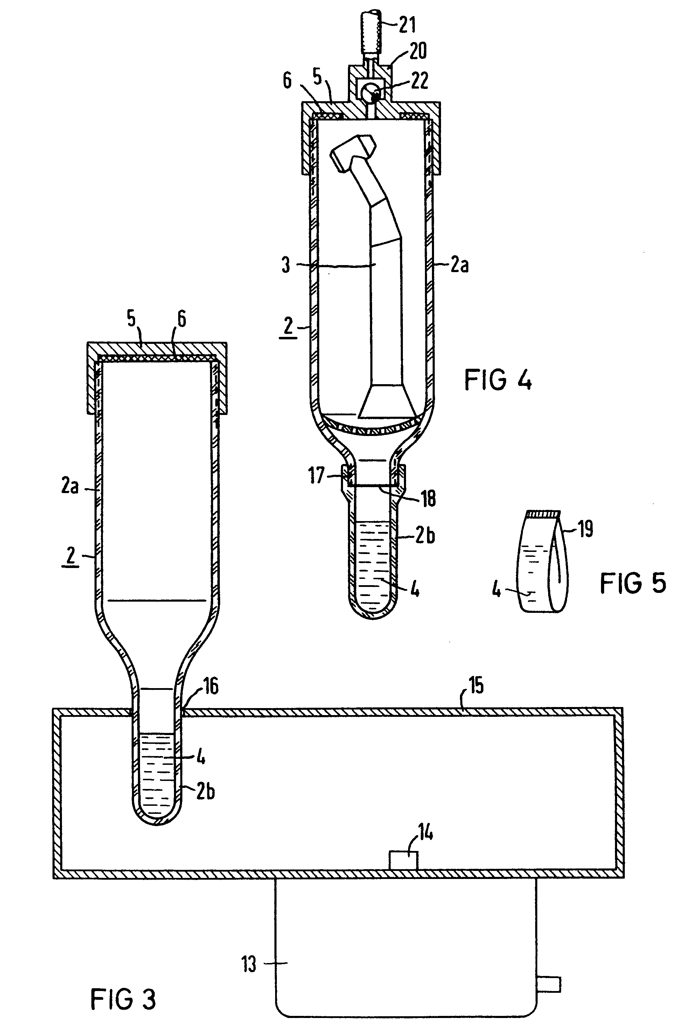

Die Fig. 3 zeigt eine andere Ausführung der erfindungsgemässen Vorrichtung. Hier ist anstelle des Infrarotstrahlers ein Mikrowellengenerator mit einem Magnetron 13 vorgesehen. Das Magnetron 13 gibt Mikrowellenstrahlung über einen Auskoppelstutzen 14 in ein Gehäuse 15, welches als Hohlresonator dient. Dieser Hohlresonator ist im Bereich der höchsten Leistungsdichte, die empirisch ermittelt wird, mit einer Bohrung 16 von etwa 8 bis 10 mm Durchmesser versehen, durch die hindurch in den Hohlresonator der Behälterabschnitt 2b mit dem Reaktionsmedium ragt. Unter Einwirkung der Mikrowellenenergie siedet das Reaktionsmedium und geht in Dampfphase über. Am noch kalten Sterilisationsgut und an den Innenwandungen des Behälters 2 kondensiert dann das Medium wieder und tropft schliesslich wieder zurück in den Behälterabschnitt 2b. Der gesamte Behälter 2 ist im Gehäuse 15 also leicht herausnehmbar gehaltert.

Das Gehäuse 15 kann mehrere Öffnungen 16 zur Aufnahme mehrerer Aufnahmebehälter 2 aufweisen, so dass mehrere Behälter gleichzeitig beaufschlagt werden können. Der Behälter 2 kann so ausgebildet sein, dass er als Aufbewahrungsbehälter dienen kann; denkbar ist jedoch auch, ihn ortsfest am Gehäuse zu befestigen und das zu sterilisierende Gut in gasdurchlässige Folienschläuche eingeschweisst in den Behälter zu geben.

Die Leistung des Magnetron beträgt etwa 500 W, bezogen auf das eingangs bereits angegebene Aufnahmevolumen für den Behälter. Die Schaltung kann vorteilhafterweise so gestaltet sein, dass die Mikrowellenleistung nach Erreichen der restlosen Verdampfung des Reaktionsmediums reduziert, z.B. auf die Hälfte herabgesetzt wird. Eine hierzu erforderliche Abtastung kann entweder fotoelektrisch erfolgen oder über den Dampfdruck.

Eine andere vorteilhafte Ausgestaltung des Behälters 2 zur Aufnahme des zu sterilisierenden Gegenstandes und der Reaktionsflüssigkeit ist in Fig. 4 dargestellt. Die Behälterabschnitte 2a, 2b sind dort durch eine Schraubverbindung 17 miteinander verbunden. Dazwischen angeordnet und durch die Schraubverbindung 17 eingespannt ist eine Trennfolie 18. Das Material der Trennfolie ist so gewählt, dass bei einem bestimmten Dampfdruck, in der Regel zwischen 1,5 und 5 bar, die Folie platzt und der Dampf sich dann explosionsartig im Behälterabschnitt 2a, in dem sich das Instrument 3 befindet, ausbreiten kann. Ein wesentlicher Vorteil dieser Lösung ist, dass eine äusserst kurze, aber sehr kräftige Beaufschlagung mit einer sehr hohen kinetischen Energie gegeben ist, wodurch, wie Versuche gezeigt haben, eine besonders gute Keimabtötung bewirkt wird.

Anstelle die Reaktionsflüssigkeit 4 in den Behälterabschnitt 2b einzufüllen, kann auch das Reaktionsmedium in gleichmässigen Portionen in Beuteln abgepackt werden. Fig. 5 zeigt einen solchen Beutel. Der Beutel 19 besteht aus durchsichtigem Plastik, hat ein Volumen von etwa 2 bis 10 cm³ und ist bis zu etwa 60% mit der Reaktionsflüssigkeit 4 gefüllt. Zweckmässigerweise ist der Beutel folienverschweisst. Der Beutel wird vor Verschraubung der beiden Behälterabschnitte 2a, 2b in den unteren Abschnitt 2b (Flaschenhals) eingelegt. Hierbei entfällt natürlich das Einlegen der zuvor beschriebenen Trennfolie 18.

Besonders wirksam ist eine Sterilisation, wenn der obere Behälterabschnitt 2a nach Einlegen des Instruments 3 und Verschliessen des Behälterabschnittes mittels des Deckels 5 über einen Anschluss 20 und eine Leitung 21 an eine nicht dargestellte Unterdruckanlage angeschlossen wird und im Behälterabschnitt 2a ein Unterdruck von etwa 50 Torr erzeugt wird. Dies kann in einfacher Weise z.B. mittels einer Wasser- oder Luftstrahlpumpe erfolgen. Ein mit 22 bezeichnetes Rückschlagventil sorgt dafür, dass nach Abnehmen der Anschlussleitung 21 der Unterdruck im Behälterraum erhalten bleibt.

Das zuvor beschriebene Verfahren des schlagartigen Ausbreitens des Reaktionsmediums in der Dampfphase, wenn ein bestimmter Dampfdruck erreicht wird, in Verbindung mit dem im Behälterabschnitt 2 erzeugten leichten Unterdruck, führt zu einer besonders intensiven Sterilisation, insbesondere weil der sich schlagartig ausbreitende Dampf durch den vorhandenen Unterdruck praktisch in jede kleinste Spalte „eingesaugt" wird.

Die erfindungsgemäss vorgestellte Vorrichtung kann wegen ihres raumsparenden Aufbaus überall in der medizinischen Praxis, z.B. in bakteriologischen, hygienischen oder chemischen Labors, eingesetzt werden; eine besonders vorteilhafte Anwendung liegt in der zahnärztlichen Praxis.

## Patentansprüche

1. Vorrichtung zum Sterilisieren von medizinischen, insbesondere zahnmedizinischen Gegenständen (3), wie Handinstrumenten od. dgl., unter Verwendung eines Strahlers (8) mit hoher Energieabstrahlung, vorzugsweise eines IR- oder Mikrowellenstrahlers und eines flüssigen, dampfbildenden Reaktionsmediums (4), dadurch gekennzeichnet, dass für die zu sterilisierenden Gegenstände (3) ein relativ kleiner Aufnahmebehälter (2a) und ein mit diesem in Verbindung stehender, das Reaktionsmedium (4) aufnehmender weiterer Behälter (2b) mit einem im Vergleich zum Volumen des Aufnahmebehälters sehr kleinen Behältervolumen vorgesehen sind, und dass der weitere Behälter (2b) im Bereich höchster Energiedichte des das Reaktionsmedium (4) verdampfenden Strahlers (8, 13) angeordnet ist.

2. Vorrichtung nach Anspruch 1, dadurch gekennzeichnet, dass ein zu seinem einen Ende hin stark verjüngend ausgebildeter Behälter (2) vorgesehen ist, der einen oberen Behälterabschnitt (2a) zur Aufnahme der zu sterilisierenden Gegenstände (3) und einen mit dem oberen in Verbindung stehenden unteren Behälterabschnitt (2b) zur Aufnahme des Reaktionsmediums (4) bildet, wobei der sich verjüngende Teil des Behälters den unteren Abschnitt (2b) bildet.

3. Vorrichtung nach Anspruch 2, dadurch gekennzeichnet, dass der Behälter (2) flaschenartig geformt ist, wobei der flaschenhalsförmige Teil den untenliegenden Behälterabschnitt (2b) zur Aufnahme des Reaktionsmediums (4) bildet.

4. Vorrichtung nach einem der Ansprüche 1 bis 3, dadurch gekennzeichnet, dass Aufnahmebehälter (2a) und weiterer Behälter (2b) eine in einem den Strahler (8, 13) aufnehmenden Gehäuse (1, 15) leicht entnehmbar gehalterte Einheit bilden.

5. Vorrichtung nach Anspruch 4, dadurch gekennzeichnet, dass das Gehäuse (1, 15) mehrere deckseitige Öffnungen (16) zur Aufnahme der das Reaktionsmedium aufnehmenden Behälterabschnitte (2b) von mehreren Behältern (2) enthält.

6. Vorrichtung nach einem der Ansprüche 1 bis 5, dadurch gekennzeichnet, dass der Aufnahmebehälter (2a) zur Aufnahme von im wesentlichen nur einem Handinstrument (3) ausgebildet ist.

7. Vorrichtung nach einem der Ansprüche 1 bis 6, dadurch gekennzeichnet, dass als Strahler ein IR-Strahler (8) vorgesehen ist und der das Reaktionsmedium aufnehmende Behälterabschnitt (2b) im ersten Brennpunkt (10) eines elliptischen Reflektors (9) liegt, in dessen zweiten Brennpunkt (11) der IR-Strahler (8) angeordnet ist.

8. Vorrichtung nach Anspruch 7, dadurch gekennzeichnet, dass der Infrarotstrahler (8) stabförmig ausgebildet ist.

9. Vorrichtung nach Anspruch 7, dadurch gekennzeichnet, dass der Reflektor (9) eine Bauhöhe aufweist, die zumindest der Bauhöhe des Behälterabschnittes (2b) zur Aufnahme des Reaktionsmediums entspricht.

10. Vorrichtung nach Anspruch 7, dadurch gekennzeichnet, dass die Innenseite des Reflektors (9) mit einem gut reflektierenden Belag (12), z.B. mit einer Goldbeschichtung, versehen ist.

11. Vorrichtung nach einem der Ansprüche 7 bis 10, dadurch gekennzeichnet, dass der Reflektor (9) aus dünnwandigem Material mit geringer Wärmekapazität hergestellt ist.

12. Vorrichtung nach einem der Ansprüche 1 bis 11, dadurch gekennzeichnet, dass wenigstens der zur Aufnahme des Reaktionsmediums dienende Behälterabschnitt (2b) aus einem Werkstoff mit guter Infrarotdurchlässigkeit, vorzugsweise aus Glas, besteht.

13. Vorrichtung nach einem der Ansprüche 1 bis 12, dadurch gekennzeichnet, dass als Reaktionsmedium (4) eine Flüssigkeit oder ein Flüssigkeitsgemisch verwendet ist, das eine hohe Infrarotabsorption aufweist.

14. Vorrichtung nach einem der Ansprüche 1 bis 13, dadurch gekennzeichnet, dass die Strahlung des Infrarotstrahlers (8) spektral an die Absorption des Reaktionsmediums (4) angepasst ist.

15. Vorrichtung nach einem der Ansprüche 1 bis 6, dadurch gekennzeichnet, dass als Strahler ein Mikrowellengenerator (13) vorgesehen ist, der über einen Auskoppelstutzen (14) mit einem als Hohlresonator dienenden Gehäuse (15) verbunden ist, in das der das Reaktionsmedium (4) aufnehmende Behälterabschnitt (2b) eintaucht.

16. Vorrichtung nach einem der Ansprüche 1 bis 15, dadurch gekennzeichnet, dass die beiden Behälterabschnitte (2a, 2b) durch einen Schnellverschluss, z.B. eine Verschraubung (17) miteinander verbunden sind und dazwischen eine die beiden Räume gegeneinander verschliessende Trennfolie (18) angeordnet ist, welche so ausgebildet ist, dass sie bei einem definierten Dampfdruck des Reaktionsmediums (4) im Behälterabschnitt (2b), vorzugsweise bei einem Dampfdruck von 1,5 bis 5 bar, platzt und so einen explosionsartigen Übertritt des Reaktionsmediums (4) vom Behälterabschnitt (2b) in den Behälterabschnitt (2a) ermöglicht.

17. Vorrichtung nach einem der Ansprüche 1 bis 15, dadurch gekennzeichnet, dass das Reaktionsmedium (4) in einem Beutel (19) eingefüllt ist, der in den Behälterabschnitt (2b) eingelegt wird, und dass der Beutel (19) so gestaltet ist, dass er bei einem definierten Dampfdruck, vorzugsweise bei 1,5 bis 5 bar, platzt und so einen explosionsartigen Übertritt des Reaktionsmediums (4) vom Behälterabschnitt (2b) in den Behälterabschnitt (2a) ermöglicht.

18. Vorrichtung nach einem der Ansprüche 1 bis 17, dadurch gekennzeichnet, dass der die zu sterilisierenden Gegenstände (3) aufnehmende Behälterabschnitt (2a) mit einem Anschluss (20) an eine Unterdruckanlage versehen ist, mit dem im Behälterraum (2a) ein Unterdruck von etwa 1 bis 200 Torr erzeugt werden kann.

## Claims

1. Apparatus for sterilising medical articles, in particular dental articles (3), such as manual instruments or the like, using a radiator (8) which emits high energy radiation, preferably an IR- or microwave-radiator, and a liquid, vapour-forming reaction medium (4), characterised in that there is provided a relatively small receiving container (2) for the articles (3) which are to be sterilised and a further container (2b) which is connected to the receiving container, accommodates the reaction medium (4), and has a very small container volume in comparison with the volume of the receiving container; and that the further container (2b) is arranged in the region of highest energy density of the radiator (8, 13) which serves to vaporize the reaction medium (4).

2. Apparatus according to Claim 1, characterised in that a container (2) is provided which narrows strongly towards one of its ends and which has an upper container section (2a) which accommodates the articles (3) to be sterilised, and a lower container section (2b) which is connected to the upper section and which serves to accommodate the reaction medium (4), the narrowing part of the container forming the lower section (2b).

3. Apparatus according to Claim 2, characterised in that the container (2) is bottle-shaped, the neck of the bottle forming the lower container section (2b) which serves to accommodate the reaction medium (4).

4. Apparatus according to one of Claims 1 to 3, characterised in that the receiving container (2a) and the further container (2b) form a single unit which is mounted so as to be easily removable, in a housing (1, 15) which accommodates the radiator (8, 13).

5. Apparatus according to Claim 4, characterised in that the top part of the housing (1, 15) contains a plurality of openings (16) for the reception of the container sections (2b) which accommodate the reaction medium of a plurality of containers (2).

6. Apparatus according to one of Claims 1 to 5, characterised in that the receiving container (2a) is designed for the reception of essentially only one manual instrument (3).

7. Apparatus according to one of Claims 1 to 6, characterised in that as the radiator, an IR-radiator (8) is provided, and the container section (2b) which accommodates the reaction medium is arranged at the first focal point (10) of an elliptical reflector (9) at the second focal point (11) of which the IR-radiator (8) is arranged.

8. Apparatus according to Claim 7, characterised in that the infra-red radiator (8) is rod-shaped.

9. Apparatus according to Claim 7, characterised in that the height of the reflector (9) at least corresponds to the height of the container section (2b) which serves to accommodate the reaction medium.

10. Apparatus according to Claim 7, characterised in that the inner side of the reflector (9) is provided with a highly reflective coating (12), e.g. a gold coating.

11. Apparatus according to one of Claims 7 to 10, characterised in that the reflector (9) is formed from thin-walled material having a low heat capacity.

12. Apparatus according to one of Claims 1 to 11, characterised in that at least that container section (2b) which serves to accommodate the reaction medium consists of a material which has good infra-red permeability, preferably glass.

13. Apparatus according to one of Claims 1 to 12, characterised in that the reaction medium (4) consists of a liquid or a mixture of liquids which has a high infra-red absorption.

14. Apparatus according to one of Claims 1 to 13, characterised in that the radiation of the infra-red radiator (8) is spectrally matched to the absorption of the reaction medium (4).

15. Apparatus according to one of Claims 1 to 6, characterised in that the radiator consists of a microwave generator (13) which is connected by way of an output coupling (14) to a housing (15) which serves as a resonant chamber and into which the container section (2b) which accommodates the reaction medium (4) extends.

16. Apparatus according to one of Claims 1 to 15, characterised in that the two container sections (2a, 2b) are connected to one another by a high-speed closure means, e.g. a screw (17), and between them a separating foil (18) is arranged which seals the two chambers from one another and is such that, when the reaction medium (4) in the container section (2b) reaches a predetermined vapour pressure, preferably a vapour pressure of 1.5 to 5 bar, it shatters and thus makes possible an explosive passage of the reaction medium (4) from the container section (2b) into the container section (2a).

17. Apparatus according to one of Claims 1 to 15, characterised in that the reaction medium (4) is filled into a bag (19) which is inserted into the container section (2b), and that the bag (19) is such that, at a determinate vapour pressure, preferably 1.5 to 5 bar, it shatters and thus makes possible an explosive passage of the reaction medium (4) from the container section (2b) into the container section (2a).

18. Apparatus according to one of Claims 1 to 17, characterised in that the container section (2a) which accommodates the articles (3) which are to be sterilised is provided with a connection (20) to a reduced pressure system, by means of which a reduced pressure of about 1 to 200 Torr can be produced in the container chamber (2a).

## Revendications

1. Dispositif pour la stérilisation d'objets médicaux, notamment d'objets de dentisterie (3), tels que des instruments à main ou analogues, moyennant l'utilisation d'un radiateur (8) produisant un rayonnement d'énergie intense, de préférence un radiateur à infrarouge ou à microondes, et d'un milieu réactionnel liquide (4) formant une vapeur, caractérisé par le fait qu'il est prévu pour les objets (3) devant être stérilisés, un récipient relativement petit d'entreposage (2a) et un autre récipient (2b) relié au précédent et recevant le milieu réactionnel (4) et possédant un volume très faible par rapport au volume du récipient d'entreposage, et que l'autre récipient (2b) est disposé dans la région de densité d'énergie maximale du radiateur (8, 13) provoquant l'évaporation du milieu réactionnel (4).

2. Dispositif suivant la revendication 1, caractérisé par le fait qu'il est prévu un récipient (2) se rétrécissant fortement vers l'une de ses extrémités et qui comporte une section supérieure (2a) servant à recevoir les objets (3) devant être stérilisés et une section inférieure (2b) reliée à la section supérieure et servant à recevoir le milieu réactionnel (4), la partie rétrécie du récipient constituant la section inférieure (2b).

3. Dispositif suivant la revendication 2, caractérisé par le fait que le récipient (2) est réalisé en forme de bouteille dont la partie en forme de goulot constitue la section inférieure (2b) du récipient destinée à recevoir le milieu réactionnel (4).

4. Dispositif suivant l'une des revendications 1 à 3, caractérisé par le fait que le récipient (2a) d'entreposage et l'autre récipient (2b) forment une unité maintenue de façon à pouvoir en être aisément retirée dans un boîtier (1, 15) logeant le radiateur (8, 13).

5. Dispositif suivant la revendication 4, caractérisé par le fait que le boîtier (1, 15) comporte plusieurs ouvertures (16) situées à la partie supérieure et servant à recevoir les sections (2b), logeant le milieu réactionnel, de plusieurs récipients (2).

6. Dispositif suivant l'une des revendications 1 à 5, caractérisé par le fait que le récipient d'entreposage (2a) est agencé de manière à recevoir essentiellement un seul instrument à main (3).

7. Dispositif suivant l'une des revendications 1 à 6, caractérisé par le fait qu'il est prévu comme radiateur un radiateur à infrarouge (8) et que la section (2b) du récipient recevant le milieu réactionnel est située au premier foyer (10) d'un réflecteur elliptique (9), au second foyer (11) duquel se trouve disposé le radiateur à infrarouge (8).

8. Dispositif suivant la revendication 7, caractérisé par le fait que le radiateur à infrarouge (7) est réalisé en forme de barreau.

9. Dispositif suivant la revendication 7, caractérisé par le fait que le réflecteur (9) possède une hauteur hors tout qui correspond au moins à la hauteur hors tout de la section (2b) du récipient servant à recevoir le milieu réactionnel.

10. Dispositif suivant la revendication 7, caractérisé par le fait que la face intérieure du réflecteur (9) est munie d'un revêtement (12) possédant de bonnes propriétés de réflexion, par exemple un revêtement d'or.

11. Dispositif suivant l'une des revendications 7 à 10, caractérisé par le fait que le réflecteur (9) est réalisé en un matériau à paroi mince possédant une faible capacité calorifique.

12. Dispositif suivant l'une des revendications 1 à 11, caractérisé par le fait qu'au moins la section (2b) du récipient servant à recevoir le milieu réactionnel est constituée en un matériau possédant une bonne perméabilité aux infrarouges, de préférence du verre.

13. Dispositif suivant l'une des revendications 1 à 12, caractérisé par le fait qu'on utilise comme milieu réactionnel (4) un liquide ou un mélange de liquides, qui réalise une forte absorption des infrarouges.

14. Dispositif suivant l'une des revendications 1 à 13, caractérisé par le fait que le rayonnement du radiateur à infrarouge (8) est adapté, du point de vue spectral, à l'absorption du milieu réactionnel (4).

15. Dispositif suivant l'une des revendications 1 à 6, caractérisé par le fait qu'il est prévu comme radiateur un générateur à micro-ondes (13) qui est relié par l'intermédiaire d'un tube de découplage (14) à un boîtier (15) servant de résonateur à cavité et dans lequel pénètre la section (2b) du récipient recevant le milieu réactionnel (4).

16. Dispositif suivant l'une des revendications 1 à 15, caractérisé par le fait que les deux sections (2a, 2b) du récipient sont reliées entre elles par un dispositif de fermeture rapide, par exemple par une liaison vissée (17) et qu'entre ces sections se trouve disposée une feuille de séparation (18) séparant l'un de l'autre les deux espaces et qui est agencée de telle manière que pour une pression de vapeur définie du milieu réactionnel (4) dans la section (2b) du récipient, de préférence pour une pression de vapeur comprise entre 1,5 et 5 bar, cette feuille éclate et permet ainsi un passage, à la manière d'une explosion, du milieu réactionnel (4) depuis la section (2b) dans la section (2a) du récipient.

17. Dispositif suivant l'une des revendications 1 à 15, caractérisé par le fait que le milieu réactionnel (4) est introduit dans un sachet (19) qui est placé dans la section (2b) du récipient, et que le sachet (19) est conçu de telle sorte que pour une pression de vapeur définie, de préférence comprise entre 1,5 et 5 bar, il éclate et permet ainsi un passage, à la manière d'une explosion, du milieu réactionnel (4) depuis la section (2b) dans la section (2a) du récipient.

18. Dispositif suivant l'une des revendications 1 à 17, caractérisé par le fait que la section (2a) du récipient, qui reçoit les objets (3) devant être stérilisés, est munie d'un raccord (20) de liaison à une installation à dépression, grâce auquel on peut obtenir dans l'espace (2a) du récipient une dépression comprise entre environ 1 à 200 Torr.

FIG 1

FIG 2

FIG 4

FIG 5

FIG 3